Europäisches Patentamt

European Patent Office

Office européen des brevets

(11) Veröffentlichungsnummer: **0 304 700 B1**

(12) **EUROPÄISCHE PATENTSCHRIFT**

(45) Veröffentlichungstag der Patentschrift: **05.02.92**

(51) Int. Cl.⁵: **A61M 37/00**

(21) Anmeldenummer: **88112758.3**

(22) Anmeldetag: **05.08.88**

(54) **Vorrichtung zur Applikation von Implantaten.**

(30) Priorität: **11.08.87 DE 3726654**
**26.01.88 DE 3802158**

(43) Veröffentlichungstag der Anmeldung:
**01.03.89 Patentblatt 89/09**

(45) Bekanntmachung des Hinweises auf die
Patenterteilung:
**05.02.92 Patentblatt 92/06**

(84) Benannte Vertragsstaaten:
**AT BE CH DE ES FR GB GR IT LI LU NL SE**

(56) Entgegenhaltungen:
**DE-A- 3 419 876**
**US-A- 3 921 632**
**US-A- 4 308 859**

(73) Patentinhaber: **HOECHST AKTIENGESELL-
SCHAFT**
**Postfach 80 03 20**
**W-6230 Frankfurt am Main 80(DE)**

(72) Erfinder: **Sandow, Jürgen Kurt, Dr.**
**Am Haideplacken 22**
**W-6240 Königstein/Taunus(DE)**
Erfinder: **De Felice, Wilfried**
**Im Stückes 26**
**W-6233 Kelkheim (Taunus)(DE)**
Erfinder: **Pajunk, Horst**
**Am Holzplatz 7**
**W-7716 Geisingen(DE)**
Erfinder: **Pajunk, Heinrich**
**Am Holzplatz 5**
**W-7716 Geisingen(DE)**

**Beschreibung**

Gegenstand der Erfindung ist eine Vorrichtung zur Injektion von Implantaten, bevorzugt stäbchenförmigen Implantaten (sogenannten Rods), die zur Langzeitbehandlung von Erkrankungen dienen.

Die Applikation solcher Implantate hat in letzter Zeit Bedeutung erlangt, da Kunststoffe entwickelt wurden, die in vivo kontrolliert abgebaut werden und somit den matrixartig eingeschlossenen oder chemisch an sie gebundenen Wirkstoff über einen längeren Zeitraum an den Organismus abgeben (Wirkstoffdepotformen).

Eine moderne Arzneitherapie kann auf diese Darreichungsformen, die eine kontrollierte Abgaberate der Wirkstoffe mit einer hohen Biokompatibilität des Depots vereinigen, nicht mehr verzichten. Eine solche langandauernde kontrollierte Wirkstoffabgabe ist wegen der zunehmenden Bedeutung chronischer Erkrankungen und langzeitorientierter Therapiekonzepte in Human- und Veterinärmedizin von großer Aktualität.

Vorrichtungen zur Applikation der meist als zylindrische Stäbchen (Rods) ausgebildeten Wirkstoffdepot-Implantaten sind bereits bekannt. Sie bestehen meist aus einem Wirkstoffbehälter, in dem sich das "Rod" befindet, einer daran angesetzten Injektionskanüle und einem Stößel zur Einbringung der Wirkstoffzubereitung durch Vorschieben in das Subkutangewebe.

In der deutschen Offenlegungsschrift 34 19 876 ist eine mechanisch relativ aufwendige Vorrichtung zur subkutanen Applikation von Depotkörpern beschrieben, bei der der Depotkörper sich unmittelbar vor der spitzen Kanülenöffnung befindet. Bei der Applikation wird die Kanüle in das Subkutangewebe eingeführt. Mittels eines verschiebbaren Glieds wird dann die Nadel aus dem Gewebe herausgezogen, wobei eine feststehende Stößelstange, die an dem Depotkörper anliegt, dafür sorgt, daß sich allein die Kanüle konzentrisch um Depotkörper und Stößelstange zurückzieht und der Depotkörper im Gewebe verbleibt. Der Depotkörper wird also nicht in das Subkutangewebe hineingeschoben, sondern wird im Gewebe "freigelegt", indem sich die hohlzylindrische Kanüle über ihn hinwegzieht.

Aus der Produktinformation zum (R)ZOLADEX-Depot (ICI) ist eine Vorrichtung zur Applikation von Implantaten bekannt, bei der der Wirkstoffbehälter ein durchsichtiges Mittelteil (Fenster) enthält, so daß man erkennen kann, ob sich ein "Rod" in der Vorrichtung befindet oder nicht.

Anwendungstechnisch weist der von ICI beschriebene Injektor jedoch eine Reihe von Nachteilen auf:

a) Zwar befindet sich die gesamte Applikationsvorrichtung steril verpackt in einer Blisterpak-

kung; einmal geöffnet muß die Vorrichtung aber möglichst schnell verwendet werden, da anderenfalls die Sterilität nicht mehr gewährleistet ist. Dabei wird unterstellt, daß die Kanüle noch mit einer Schutzkappe versehen ist, die Stößelstange jedoch bleibt im "ausgepackten" Zustand nicht steril, was dann von Nachteil sein kann, wenn die Stößelstange mehrfach hin und hergeschoben wird, wobei die Sterilität des Wirkstoffzylinders nicht mehr gewährleistet werden kann.

b) Es besteht die Gefahr, daß beim Einführen der Kanüle in das Subkutangewebe, vorzeitig und unbeabsichtigt die Stößelstange betätigt wird.

Diese Nachteile werden durch die nachfolgend aufgeführten konstruktiven Änderungen gegenüber dem oben beschriebenen ICI-Injektor vermieden.

Die Erfindung betrifft dementsprechend:
Eine Vorrichtung zur Injektion von Implantaten, bestehend aus einem Wirkstoffbehälter, einer Kanüle und einer Stößelstange, dadurch gekennzeichnet, daß der zylindrische Stößelkanal, der durch den Wirkstoffbehälter (Injektorkörper) verläuft, einen konzentrisch um den Stößelkanal verlaufenden, flexiblen O-Ring enthält, der den Innendurchmesser des zylindrischen Stößelkanals geringfügig verkleinert und dadurch, daß die Stößelstange eine konzentrische Nut aufweist und dadurch, daß die Vorrichtung weiterhin mit einer Schutzkappe sowohl für die Kanüle als auch für die Stößelstange versehen ist und dadurch, daß zumindest der Wirkstoffbehälter aus durchsichtigem Kunststoff gefertigt ist.

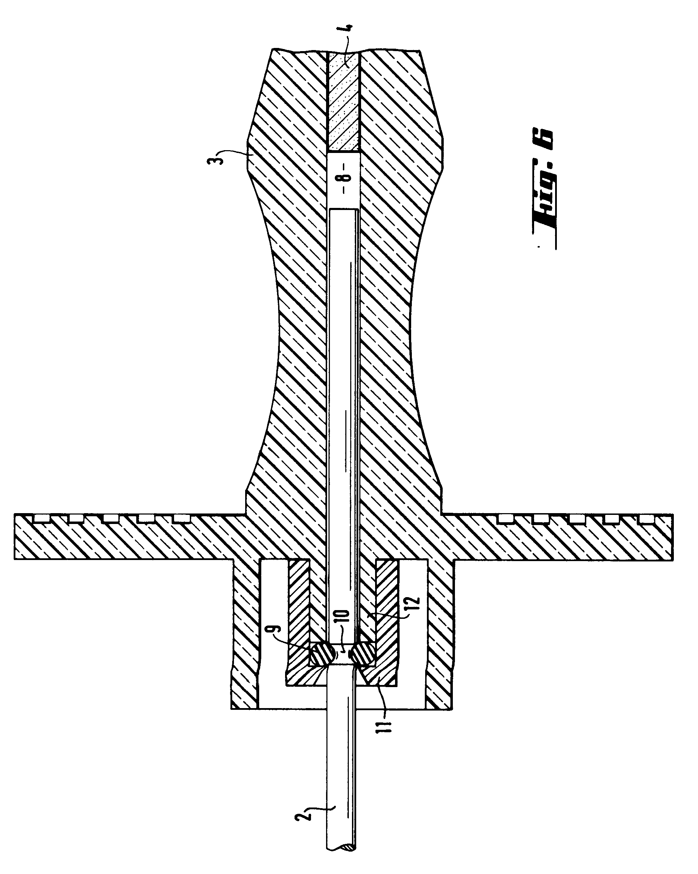

In den Figuren 1 - 5 ist die Erfindung näher erläutert.

In Figur 1 ist die Vorrichtung ohne Schutzkappen für Stößelstange und Kanüle dargestellt. In dem Injektorkörper (3) befindet sich der Wirkstoffzylinder (4) vor der Öffnung bzw. schon teilweise in der Kanüle (5). Das Vorschieben des Wirkstoffzylinders (4) in das Subkutangewebe erfolgt mittels der Stößelstange (2), an dessen der Kanüle entgegengesetzten Ende sich der Stößelknopf (1) befindet. Der einstückige Injektorkörper (3) ist aus durchsichtigem Kunststoff gefertigt. Die Stößelstange und die Kanüle sind aus Metall gefertigt.

In der Figur 2 ist die Schutzkappe (6) für die Stößelstange und in der Figur 3 die Schutzkappe (7) für die Kanüle dargestellt. Beide Teile (6 und 7) können bevorzugt ebenfalls aus durchsichtigem Kunststoff gefertigt sein.

In Figur 4 ist die gesamte Vorrichtung mit aufgesteckten Schutzkappen (6 und 7) sowohl für die Stößelstange als auch für die Kanüle dargestellt. Figur 5 zeigt die Stößelstange (2) mit der Nut (10).

Figur 6 zeigt in vergrößerter Detaildarstellung den Mittelbereich der Figur 1.

Erfindungsgemäß enthält der Wirkstoffbehälter (3) einen konzentrisch um den Stößelkanal (B) verlaufenden, flexiblen O-Ring (9), der den Innendurchmesser des zylindrischen Stößelkanals geringfügig verkleinert. Diese Verkleinerung des Innendurchmessers beträgt 5 - 40 %, bevorzugt 10 - 30 %, insbesondere 10 - 20 %, von dem Durchmesser des Stößelkanals. Die Stößelstange (2) selbst besitzt eine ringförmige (konzentrisch um die Stößelstange) verlaufende Nut (10), deren Tiefe bevorzugt 2,5 - 25 %, besonders bevorzugt 5 - 20 %, ganz besonders bevorzugt 7,5 - 12,5 % von dem Durchmesser der Stößelstange beträgt.

Die restlichen Ausgestaltungen der Vorrichtung, wie Griffmulden für die Kanülen- bzw. Stößelstangenschutzkappen, Zeige- und Mittelfingergriffablage oder Stößelstangenknopf, sind je nach Anwendungsbereich frei variabel und vom Fachmann den jeweiligen Anforderungen anzupassen.

Die Herstellung der Vorrichtung erfolgt nach allgemein üblichen Methoden der Kunststoffverarbeitung.

Das konzentrische Einbringen des O-Rings (9) in den Stößelkanal (8) des Wirkstoffbehälters (3) kann beispielsweise erfolgen, indem man den O-Ring auf den über den Wirkstoffbehälter herausragenden Stutzen (12) auflegt und über den O-Ring und den Stutzen eine Kappe (11) setzt, die auf dem Stutzen beispielsweise verklemmt oder verklebt wird.

Als Kunststoffe für die erfindungsgemäße Vorrichtung kommen solche in Betracht, die ungiftig, verschweißbar und sterilisierbar sind, insbesondere unempfindlich gegen Sterilisation mittels $\gamma$-Strahlen. Außerdem darf der verwendete Kunststoff mit keinem Bestandteil der verwendeten Implantate ("Rods") reagieren. Bevorzugt werden Polyamide oder Polycarbonate, insbesondere Polykohlensäureester von 4,4'-Dihydroxydiphenylalkanen, wie Polykohlensäureester des 4,4'-Dihydroxydiphenyl-2,2-propans ($^{(R)}$Makrolon, Bayer) eingesetzt. Der flexible O-Ring besteht vorzugsweise aus Polytetrafluorethylen (PTFE) oder Silikon.

Wesentliche Merkmale der Vorrichtung sind:
a) die volldurchsichtige, einstückige Ausführung, insbesondere des Injektorkörpers,
b) die Schutzkappe für die Stößelstange und
c) die Schutzvorrichtung, die das Herausfallen der Stößelstange verhindert.

Durch die volldurchsichtige Ausführung insbesondere des Injektorkörpers kann jederzeit das Vorhandensein und die Beschaffenheit des Implantats sowie die Funktion der Vorrichtung während des Implantationsvorganges durch den Anwender kontrolliert werden.

Der konzentrisch um den Stößelkanal angeordnete flexible O-Ring sorgt in Verbindung mit der in der Stößelstange befindlichen Nut dafür, daß die Stößelstange nicht unbeabsichtigt aus dem Wirkstoffbehälter herausfällt bzw. gleichzeitig mit dem Abnehmen der Stößelstangenschutzkappe aus dem Wirkstoffbehälter herausgezogen wird. Der Ring rastet gewissermaßen in der Nut ein, so daß ein weiteres unbeabsichtigtes Herausziehen der Stößelstange vermieden wird.

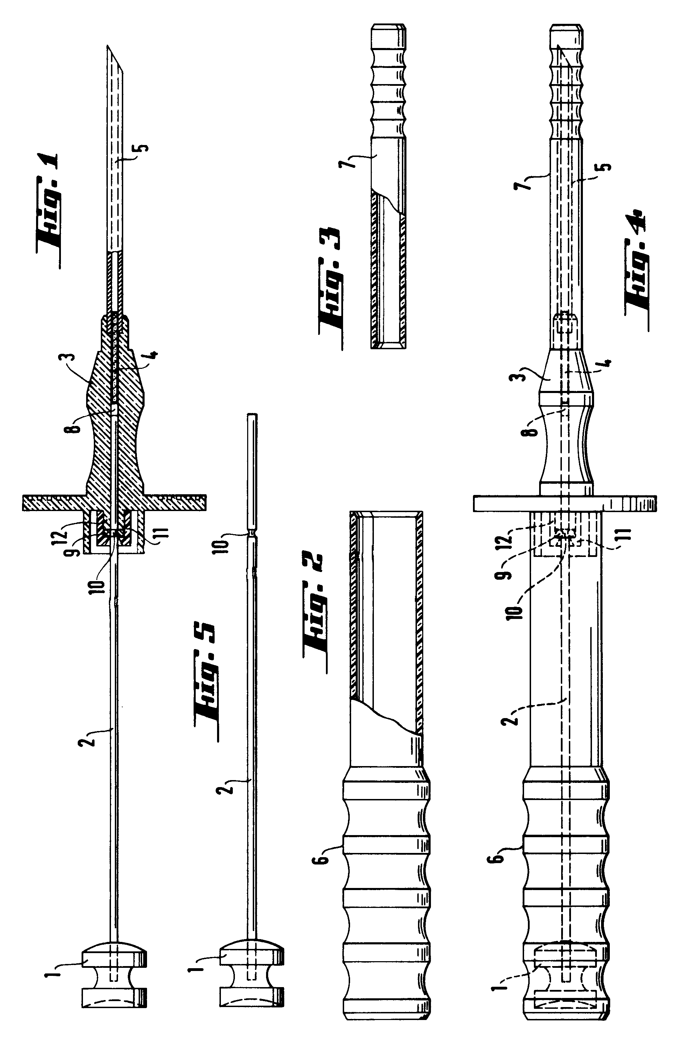

Die Schutzkappe für die Stößelstange dient in erster Linie zur Sicherung des Stößels während der Injektion, um eine unbeabsichtigte Betätigung des Stößels zu vermeiden. Darüberhinaus hält sie aber auch den von ihr abgedeckten Teil der Vorrichtung steril, so daß die erfindungsgemäße Vorrichtung auch "im ausgepackten Zustand" (s. Figur 4) weiterhin steril bleibt.

Die Implantationsvorrichtung kann vom Anwender leicht mit einer Hand bedient werden, wie dies den praktischen Erfordernissen der Behandlung entspricht. Dazu wird von der Vorrichtung wie sie in Figur 4 dargestellt ist, zunächst die Kanülenschutzkappe entfernt. Daraufhin wird die Kanüle in das Hautgewebe eingeführt. Erst jetzt wird die Schutzkappe (6) der Stößelstange entfernt und - gegebenenfalls nach teilweiser Zurückziehung der Kanüle aus dem Gewebe - das Implantat durch vollständiges Durchdrücken der Stößelstange im Gewebe placiert.

Der Anwendungsbereich betrifft die subkutane oder intramuskuläre Injektion von arzneimittelhaltigen "Rods" aus biologisch abbaubarem Polymermaterial mit den Abmessungen 0,6 mm bis 3 mm Durchmesser und einer Länge von 0,5 bis 30 mm. Die Anwendung kann auch durch Einbringung der Implantate in Gelenke oder Körperhöhlen erfolgen. Die Körperhöhle muß mit der Kanüle zugänglich sein und es ermöglichen, daß der Wirkstoff abtransportiert wird. Beispielsweise seien hier genannt: Stirnhöhle, Brusthöhle, Bauchhöhle oder Zysten oder durch Entzündungen oder andere krankhafte Prozesse entstandene Hohlräume.

**Patentansprüche**

1. Vorrichtung zur Injektion vom Implantaten, bestehend aus einem Wirkstoffbehälter, einer Kanüle und einer Stößelstange, dadurch gekennzeichnet, daß der zylindrische Stößelkanal, der durch den Wirkstoffbehälter (Injektorkörper) verläuft, einen konzentrisch um den Stößelkanal verlaufenden, flexiblen O-Ring enthält, der den Innendurchmesser des zylindrischen Stößelkanals geringfügig verkleinert und dadurch, daß die Stößelstange eine konzentrische Nut aufweist und dadurch, daß die Vorrichtung weiterhin mit einer Schutzkappe sowohl für die Kanüle als auch für die Stößelstange versehen ist und dadurch, daß zumindest der Wirkstoffbehälter aus durchsichtigem Kunststoff gefertigt ist.

2. Vorrichtung nach Anspruch 1, dadurch gekennzeichnet, daß alle Teile bis auf die Stößelstange und die Kanüle aus durchsichtigem Kunststoff gefertigt sind.

3. Vorrichtung nach Anspruch 1 oder 2, dadurch gekennzeichnet, daß der flexible O-Ring aus Polytetrafluorethylen oder Silikon gefertigt ist.

4. Vorrichtung nach einem oder mehreren der Ansprüche 1 bis 3, dadurch gekennzeichnet, daß die geringfügige Verkleinerung des Innendurchmessers des Stößelkanals durch den O-Ring 5 - 40 % des Stößelkanaldurchmessers beträgt.

5. Vorrichtung nach einem oder mehreren der Ansprüche 1 bis 4, dadurch gekennzeichnet, daß die Nut in der Stößelstange eine Tiefe von 2,5 - 25 % des Stößelstangendurchmessers hat.

6. Vorrichtung nach einem oder mehreren der Ansprüche 1 - 5, dadurch gekennzeichnet, daß die Implantate die Form zylindrischer Stäbchen haben.

**Claims**

1. A device for the injection of implants, comprising an active substance container, a cannula and a plunger rod, wherein the cylindrical plunger channel which runs through the active substance container (injector body) contains a flexible O-ring which runs concentrically around the plunger channel and slightly reduces the internal diameter of the cylindrical plunger channel, and wherein the plunger rod has a concentric groove, and wherein the device is additionally provided with a protective cap both for the cannula and for the plunger rod, and wherein at least the active substance container is fabricated from transparent plastic.

2. A device as claimed in claim 1, wherein all the parts, except the plunger rod and the cannula, are fabricated from transparent plastic.

3. A device as claimed in claim 1 or 2, wherein the flexible O-ring is fabricated from polytetrafluoroethylene or silicone.

4. A device as claimed in one or more of claims 1 to 3, wherein the slight reduction of the internal diameter of the plunger channel by the O-ring is 5 - 40% of the diameter of the plunger channel.

5. A device as claimed in one or more of claims 1 to 4, wherein the groove in the plunger rod has a depth of 2.5 - 25% of the diameter of the plunger rod.

6. A device as claimed in one or more of claims 1 to 5, wherein the implants have the form of small cylindrical rods.

**Revendications**

1. Dispositif pour l'injection d'implants, composé d'un récipient de produit actif, d'une canule et d'une tige de poussée, caractérisé en ce que le canal cylindrique (8) de poussée qui traverse le récipient (3) de produit actif (corps d'injecteur) est pourvu d'un joint torique flexible (9) qui entoure de façon concentrique le canal de poussée et réduit légèrement le diamètre intérieur du canal cylindrique de poussée, en ce que la tige (2) de poussée comporte une rainure concentrique (10), en ce que le dispositif est équipé, d'autre part, d'un capuchon protecteur (7, 6) pour la canule et pour la tige de poussée, et en ce qu'au moins le récipient (3) de produit actif est réalisé en une matière synthétique transparente.

2. Dispositif selon la revendication 1, caractérisé en ce que toutes les pièces, à l'exception de la tige de poussée et la canule, sont réalisées en matière synthétique transparente.

3. Dispositif selon la revendication 1 ou 2, caractérisé en ce que le joint torique flexible (9) est réalisé en polytétrafluoréthylène ou en silicone.

4. Dispositif selon l'une ou plusieurs des revendications 1 à 3, caractérisé en ce que la réduction légère du diamètre intérieur du canal (8) de poussée par le joint torique (9) représente 5 à 40 % du diamètre du canal de poussée.

5. Dispositif selon l'une ou plusieurs des revendications 1 à 4, caractérisé en ce que la profondeur de la rainure (10) de la tige (2) de poussée représente 2,5 à 25 % du diamètre de la tige de poussée.

6. Dispositif selon l'une ou plusieurs des revendications 1 à 5, caractérisé en ce que les implants ont la forme de bâtonnets cylindriques (4).

Fig. 1

Fig. 5

Fig. 2

Fig. 3

Fig. 4

EP 0 304 700 B1

Fig. 6